# FASCICULE DE BREVET EUROPEEN

(11) **EP 1 139 934 B1**
(45) Date de publication et mention de la délivrance du brevet: **23.08.2006**
(21) Numéro de dépôt: 99958284.4
(22) Date de dépôt: 09.12.1999
(51) Int. Cl.: A61F 2/44

(54) **PROTHESE DE DISQUE INTERVERTEBRAL**
BANDSCHEIBENPROTHESE
INTERVERTEBRAL DISC PROSTHESIS

(30) Priorité: 11.12.1998 FR 9815671
(43) Date de publication de la demande: 10.10.2001
(73) Titulaire: Stryker Spine, 33610 Cestas (FR)
(72) Inventeur: GAUCHET, Fabien, F-60800 Duvy (FR); LE COUEDIC, Régis, F-33610 Cestas (FR)
(74) Mandataire: Le Forestier, Eric
(86) Numéro de dépôt international: PCT/FR1999/003072
(87) Numéro de publication internationale: WO 2000/035384

(56) Documents cités:
- EP-A- 0 277 282
- EP-A- 0 642 775
- DE-A- 2 263 842
- FR-A- 2 723 841

## Description

L'invention concerne les prothèses de disque intervertébral.

On connaît d'après le document EP-0 277 282-A1 une prothèse de disque intervertébral comportant deux plateaux et un coussin interposé entre ceux-ci. Le coussin comporte un corps compressible délimitant une cavité remplie d'un fluide incompressible. Cette prothèse est sensiblement incompressible dans la direction axiale et autorise seulement une inclinaison relative des plateaux. Ce comportement est différent de celui d'un disque intervertébral naturel sain.

Le document FR-2 723 841 divulgue une prothèse conforme au préamble de la revendication 1.

Un but de l'invention est de fournir une prothèse de disque d'un type différent et permettant d'approcher au plus près les propriétés mécaniques d'un disque intervertébral naturel sain.

En vue de la réalisation de ce but, on prévoit selon l'invention une prothèse selon la revendication 1.

Ainsi, la compression du coussin met en oeuvre la compression du corps et celle du fluide. Les propriétés en compression du corps et du fluide pouvant être différentes, leur combinaison permet d'approcher de très près les propriétés mécaniques d'un disque intervertébral naturel sain. Notamment, lorsque le matériau du corps est convenablement choisi, on peut obtenir une courbe de la réaction mécanique à une compression du coussin en fonction d'une variation d'une dimension du coussin suivant la direction de compression, ayant une forme en hystérésis proche de celle associée à un disque naturel sain.

Avantageusement, le fluide a une pression telle qu'il est davantage compressible que le corps.

Ainsi, on peut tirer parti de cette différence pour approcher au mieux les propriétés mécaniques du disque naturel sain.

Avantageusement, le fluide comprend un gaz. Avantageusement, le coussin est agencé de sorte qu'une pression du fluide s'exerce directement sur les plateaux.

Avantageusement, le corps comprend un matériau viscoélastique, notamment du silicone.

Ainsi, on peut obtenir la courbe précitée avec une forme en hystérésis très prononcée.

Avantageusement, le corps est en contact avec les plateaux;

Avantageusement, le corps présente au moins une extrémité ayant une zone de contact avec l'un des plateaux, la prothèse étant agencée de sorte que la zone de contact a une superficie qui augmente lorsqu'on augmente une sollicitation du plateau en direction du corps.

Ainsi, pour les valeurs de compression les plus basses, la réaction mécanique de la prothèse lors de la compression du corps varie très peu en fonction du changement de dimension du coussin suivant la direction de compression. Autrement dit, la courbe précitée est peu inclinée par rapport à l'horizontale pour de faibles valeurs de compression et on fournit peu d'effort en début de course. Cette propriété reproduit celle d'un disque naturel sain.

Avantageusement, la zone de contact est définie par une face du plateau et une face de l'extrémité du corps, l'une des deux faces, notamment la face du corps étant courbe et convexe et l'autre face étant plane.

Avantageusement, la zone de contact est définie par une face du plateau et une face de l'extrémité du corps, les deux faces étant courbes dans au moins une direction commune et étant respectivement concave et convexe, la face concave ayant au moins un rayon de courbure supérieur à un rayon de courbure correspondant de la face convexe.

Ainsi, cette configuration permet de mettre en oeuvre les variations de réaction mécanique telles que précitées. En outre, lorsque le corps est libre de se déplacer latéralement par rapport au plateau, comme on le verra plus loin, cette configuration assure le centrage relatif des deux faces. Par exemple, après que les deux faces ont été décalées, ces courbure permettent qu'elles se recentrent automatiquement.

Avantageusement, le corps présente au moins une extrémité en contact avec l'un des plateaux, cette extrémité étant libre de se mouvoir par rapport au plateau suivant une direction parallèle au plateau.

Avantageusement, l'extrémité est logée dans un renfoncement du plateau et apte à former une butée latérale pour le corps.

Ainsi, on peut limiter les déplacements latéraux du corps par rapport aux plateaux, voire les interdire.

Avantageusement, le coussin comporte une enceinte renfermant le fluide et agencée de sorte qu'elle a une superficie de section transversale parallèlement aux plateaux sensiblement invariable lorsque varie une compression du coussin entre les plateaux.

Avantageusement, le coussin comporte une enceinte renfermant le fluide et s'étendant en périphérie et à distance du corps.

On évite ainsi l'érosion du corps par l'enceinte au cours de son mouvement, et la dispersion de particules du corps.

Avantageusement, l'enceinte forme ressort, notamment ressort de compression.

Ainsi, l'enceinte influence la réaction du coussin lors d'une compression de celui-ci.

Avantageusement, le coussin est agencé pour présenter une courbe de réaction mécanique à une compression en fonction d'une variation d'une dimension du coussin suivant la direction de la compression, ayant une forme en hystérésis.

Avantageusement, le coussin est agencé de sorte que la réaction à la compression croît moins fortement pour des valeurs de réaction relativement faibles que pour des valeurs de réaction relativement élevées.

Avantageusement, le coussin est agencé de sorte que la réaction à la compression décroît plus fortement pour des valeurs de réaction relativement élevées que pour des valeurs de réaction relativement faibles.

Avantageusement, le coussin est agencé de sorte que la réaction à la compression a des valeurs plus élevées lorsqu'elle croît que lorsqu'elle décroît.

Avantageusement, la prothèse est destinée à la zone lombaire du rachis.

D'autres caractéristiques et avantages de l'invention apparaîtront encore dans la description suivante d'un mode préféré de réalisation et de deux variantes donnés à titre d'exemples non limitatifs. Aux dessins annexés :
- la figure 1 est une vue en perspective d'une prothèse selon l'invention ;
- la figure 2 est une vue en coupe axiale selon le plan II-II de la prothèse de la figure 1 ;
- la figure 3 est une vue à échelle agrandie d'un détail D de la figure 2 ;
- la figure 4 est une courbe indiquant la force de compression F exercée par les deux plateaux sur le coussin en fonction de la variation de la distance les séparant ;
- la figure 5 est une vue en coupe d'un détail d'une variante de réalisation de la prothèse ; et
- la figure 6 est une vue simplifiée analogue à la figure 2 montrant une deuxième variante de réalisation.

La prothèse de disque intervertébral 2 selon l'invention est ici particulièrement destinée à la zone lombaire de la colonne vertébrale du corps humain. Elle comporte deux plateaux plats 4 ayant une forme générale en haricot à hile postérieur en vue en plan. Chaque plateau 4 comporte une plaque circulaire centrale 6 et une couronne 8 s'étendant en périphérie de la plaque dans le plan de celle-ci. Au repos, les deux plateaux 4 s'étendent parallèlement l'un à l'autre, à distance et en regard l'un de l'autre avec leurs contours en coïncidence. Sur chaque plateau 4, la couronne 8 et la plaque 6 présentent chacune une gorge 27 pour la réception d'un joint 31.

La prothèse de disque 2 comporte un coussin ou partie intermédiaire 10 interposé entre les deux plateaux 4. Le coussin- comporte un corps solide compressible 12, ici en matériau viscoélastique, par exemple en silicone. Ce corps a une dureté shore-A avantageusement comprise entre 60 et 100, et ici d'environ 80. Le corps 12 a une forme de révolution autour de son axe principal 14. Il présente une face latérale cylindrique 16 et deux faces d'extrémités axiales 18 généralement perpendiculaires à l'axe 14 et de forme légèrement sphérique convexe. Chaque face 18 présente donc deux courbures identiques dans des plans perpendiculaires entre eux. Le corps 12 est disposé coaxialement avec les plaques 6. Chaque plaque 6 présente une face centrale interne plane 20 perpendiculaire à l'axe 14 et en contact avec une des extrémités axiales 18 respectives du corps 12. Ainsi, la face sphérique convexe 18 du corps est en appui sur la face plane 20 du plateau. Le corps 12 est en appui sans ancrage sur chacun des plateaux 4 de sorte qu'il est mobile par rapport à chacun de ces plateaux suivant une direction parallèle aux plateaux, c'est-à-dire perpendiculaire à l'axe principal 14. On évite ainsi la transmission de contraintes latérales de l'une à l'autre des vertèbres.

Le coussin 10 comporte en outre un soufflet 22. Le soufflet entoure le corps 14 coaxialement à celui-ci et à distance de celui-ci. Il a une forme symétrique de révolution autour de l'axe 14. Sa paroi présente de profil des ondulations 24 permettant de faire varier la longueur du soufflet 22 suivant la direction axiale 14, sans que varie sensiblement la superficie de sa section transversalement à l'axe 14. En l'espèce, ce soufflet, de même que les plateaux 4, est réalisé en titane ou alliage de titane, de sorte qu'il présente une certaine rigidité axiale et forme un ressort de compression. Il peut également être déformé suivant une direction perpendiculaire à l'axe 14 ou subir une torsion autour de l'axe 14 ou d'un axe quelconque perpendiculaire à celui-ci.

Le soufflet 22 présente à ses deux extrémités axiales des bords collés à des bords respectifs des plaques 6 s'étendant en saillie de la face interne 20. Le collage est réalisé de façon étanche de sorte que le soufflet 22 définit avec les deux plaques 6 une enceinte étanche à volume variable s'étendant autour du corps 12. Cette enceinte renferme un fluide, en l'espèce un gaz qui est ici de l'air. Les ondulations 24 les plus proches du corps 12 s'étendent à distance de celui-ci pour permettre une libre circulation du gaz de l'une à l'autre des plaques 6.

Le soufflet 22 présente en l'espèce dix convolutions, soit huit crêtes externes en plus des deux crêtes de fixation aux plateaux. Il a ici un diamètre externe d'environ 30 mm et un diamètre interne d'environ 17 mm. Sa hauteur, lorsque la prothèse est hors charge, vaut 10 mm. La paroi du soufflet peut être réalisée au moyen d'une, deux ou trois feuilles chacune de 0,1 mm d'épaisseur et dont la somme des épaisseurs forme l'épaisseur de la paroi. Le soufflet a ici en propre une raideur d'environ 1,6 N/mm.

Chaque couronne 8 comporte deux pattes 25 s'étendant en saillie d'une face externe du plateau 4 perpendiculairement au plan du plateau. Chaque patte 25 présente un orifice 27 la traversant de part en part en direction du centre de la plaque et, sur une face de la patte 25 opposée au plateau 4, une empreinte de forme sphérique. Les orifices 27 permettent la réception d'une vis à os 26 ayant une tête 28 dont une face inférieure a une forme sphérique mâle coopérant avec l'empreinte femelle de la patte 25 pour permettre une libre orientation de la vis 26 par rapport à la patte associée.

Pour réaliser un ancrage à court terme de la prothèse de disque 2 dans la colonne, on pourra ancrer les vis 26 dans le spondyle des vertèbres adjacentes au disque à remplacer.

Toutefois, on pourra prévoir un ancrage dit à long terme où, en outre, les surfaces des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces pourront être traitées pour obtenir un état de surface plus ou moins poreux, présentant des points d'ancrage pour le tissu osseux, pour assurer une meilleure interface avec ledit tissu osseux.

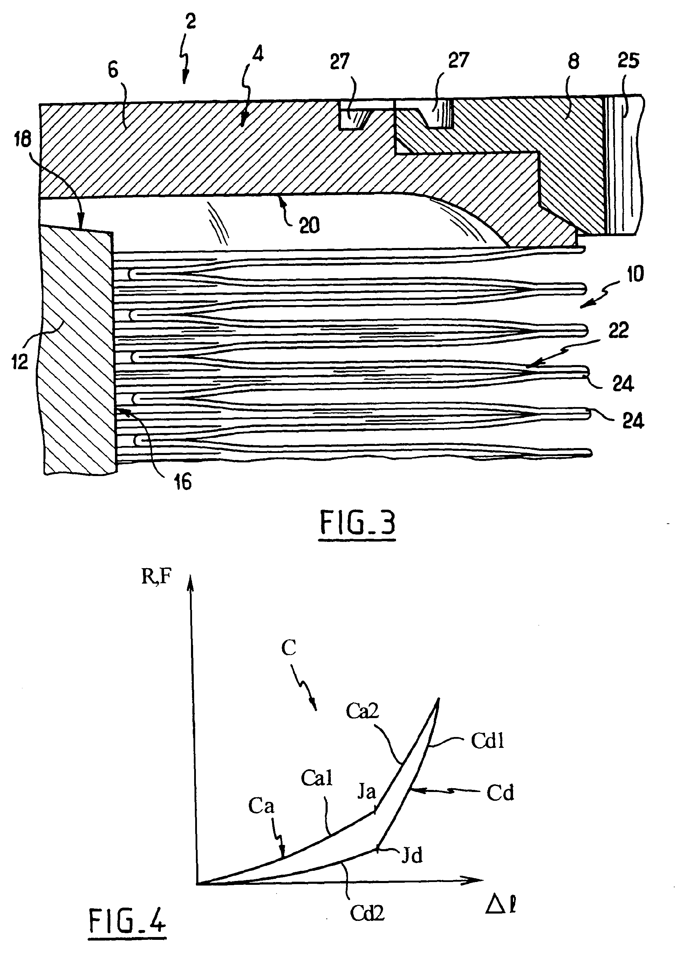

On a représenté en figure 4 l'allure de la courbe C indiquant l'intensité d'un effort de compression F exercé sur le coussin 10 (c'est-à-dire sur les deux plateaux 4) en faisant abstraction de leur déformabilité, quasi nulle, suivant la direction axiale 14, en fonction de la variation de la longueur 1 du coussin suivant la direction axiale 14 (ou encore de la distance entre les deux plateaux). Cette courbe représente également la réaction mécanique R du coussin 10 dans les mêmes conditions. Cette courbe C n'est pas linéaire. De plus, elle présente une forme en hystérésis : la courbe Ca indiquant l'augmentation de la compression F à partir de l'origine zéro étant distincte de celle Cd indiquant la diminution de la compression F jusqu'à l'origine, et s'étendant tout entière au-dessus de cette dernière. Cette forme en hystérésis prononcée est due principalement au matériau viscoélastique du corps et subsidiairement à l'association dans le coussin 10 du corps 12 et du fluide.

En outre, la courbe Ca, relative à l'augmentation de la force de compression F, présente à partir de l'origine O une portion Ca1 à faible pente, puis une portion Ca2 à pente plus forte. La courbe Cd illustrant la diminution de la compression F présente pour les valeurs les plus élevées de la force F une portion Cd1 de forte pente, puis pour les valeurs les plus basses de la force F une portion Cd2 de pente plus faible. La présence d'une portion de faible pente au voisinage de l'origine pour les courbes Ca et Cd est due principalement à la conformation des faces de contact 18, 20 du corps 12 et des plateaux 4, qui entraîne que la superficie de la zone de contact mutuel entre chaque plateau et le corps, généralement en forme de disque, augmente lorsqu'on augmente la force F. Cette augmentation se produit jusqu'à atteindre la superficie maximale de la zone de contact, lorsque toute la face 18 touche le plateau 4.

Les points de raccordement Ja et Jd forment respectivement la jonction entre les courbes Ca1 et Ca2, et Cd1 et Cd2. Sur la courbe Ca, le point Ja correspond à l'effort F pour lequel les surfaces maximales de contact entre les plateaux et le corps sont atteintes. De même, sur la courbe Cd, le point Jd correspond à l'effort pour lequel ces surfaces cessent d'être maximales.

La prothèse pourra être configurée de sorte que le point Ja corresponde à une valeur de Δ1 située entre 25% et 75% de la variation maximale de longueur envisagée pour la prothèse en utilisation.

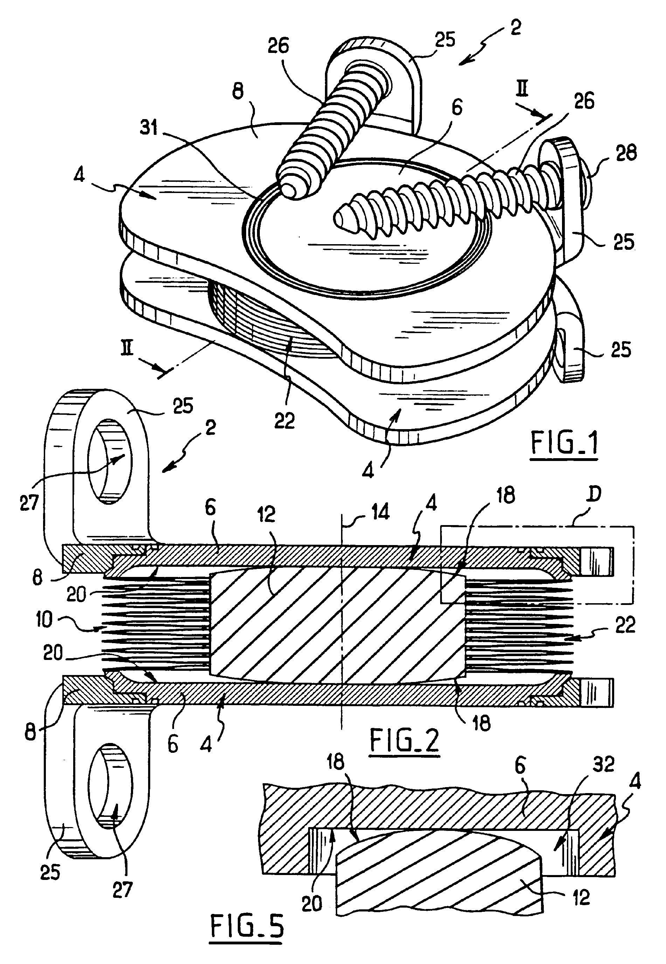

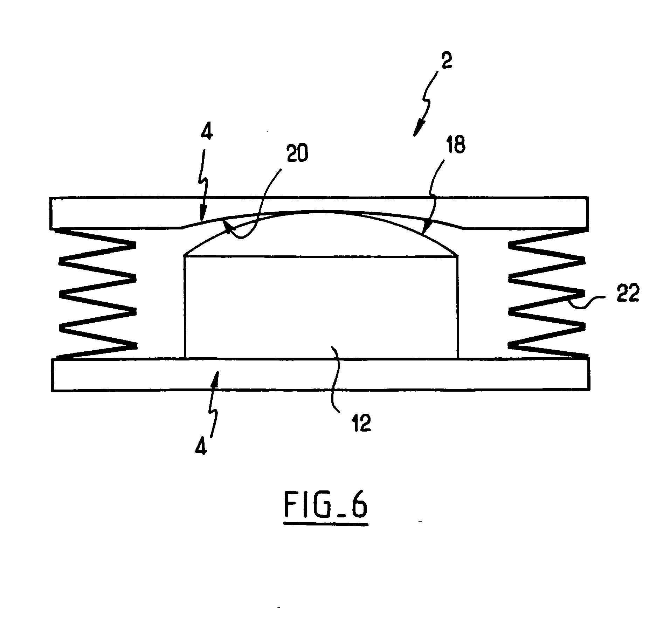

En référence à la figure 5, on pourra prévoir dans une variante de réalisation (présentant par ailleurs les autres caractéristiques de la prothèse de la figure 1) que la face 20 de chaque plateau 4 en regard du corps 12 présente un renfoncement 32, ici en « U », formant butée latérale, dans lequel vient se loger l'extrémité axiale 18 correspondante du corps. On limite ainsi à une certaine plage les déplacements relatifs latéraux du corps 12 par rapport à chaque plateau 4, voire on les interdit totalement.

Dans la variante de la figure 6, la face 20 peut être courbe et concave dans une ou deux directions, comme c'est le cas ici, et la face 18 peut être courbe et convexe dans la ou les directions correspondantes, le rayon de courbure de la face 20 étant, pour chaque direction, plus grand que celui de la face 18 dans la direction correspondante. Les deux faces 18, 20 sont ici sphériques. Les rayons de courbure des surfaces 18 et 20 seront par exemple compris entre 70 et 80 mm, et 140 et 200 mm respectivement. Un tel agencement permet d'obtenir un auto-centrage des deux faces tout en autorisant un déplacement latéral relatif du corps 12 par rapport au plateau suivant une direction quelconque perpendiculaire à une direction longitudinale du rachis.

Dans le mode de réalisation de la figure 2, les deux extrémités du corps 12 présentent une surface de contact 18 avec le plateau associé de superficie variable et le rendant- mobile latéralement par rapport au corps.

Au contraire, dans la variante de la figure 6, seule l'une des extrémités 18 du corps 12 présente cette propriété. L'autre extrémité, inférieure sur la figure 6, a une forme plane circulaire à zone de contact invariable avec le plateau associé et fixe par rapport à celui-ci.

Bien entendu, on pourra apporter à l'invention de nombreuses modifications sans sortir du cadre de celle-ci.

Le fluide pourra être un liquide, voire un mélange d'un liquide et d'un gaz, ce dernier étant par exemple faiblement soluble dans le liquide.

Le corps pourra avoir une forme elliptique en section transversale à l'axe 14.

La face interne 20 des plateaux 4 pourra être convexe, la face d'extrémité axiale 18 du corps 12 étant plane, ou concave à rayon de courbure plus grand que celui de la face 20 du plateau. Les deux faces en contact du plateau et du corps pourront être convexes.

La courbure des faces pourra être limitée à un seul plan.

On pourra mettre en oeuvre les caractéristiques relatives à l'enveloppe 22 (ressort, distance au corps 12) indépendamment des autres caractéristiques.

## Revendications

1. Prothèse de disque intervertébral (2), comportant deux plateaux (4) et un coussin (10) interposé entre les plateaux, le coussin comportant un corps solide compressible (12) et renfermant un fluide compressible **caractérisé en ce que** le fluide s'étend en périphérie du corps (12).

2. Prothèse selon la revendication 1, **caractérisée en ce que** le fluide a une pression telle qu'il est davantage compressible que le corps.

3. Prothèse selon la revendication 1 ou 2, **caractérisée en ce que** le fluide comprend un gaz.

4. Prothèse selon l'une quelconque des revendications 1 à 3, **caractérisée en ce que** le coussin (10) est agencé de sorte qu'une pression du fluide s'exerce directement sur les plateaux (4).

5. Prothèse 4 selon l'une quelconque des revendications 1 à 4, **caractérisée en ce que** le corps (12) comprend un matériau viscoélastique.

6. Prothèse selon la revendication 5, **caractérisée en ce que** le matériau est du silicone.

7. Prothèse selon l'une quelconque des revendications 1 à 6, **caractérisée en ce que** le corps (12) présente au moins une extrémité (18) ayant une zone de contact avec l'un des plateaux (4), la prothèse étant agencée de sorte que la zone de contact a une superficie qui augmente lorsqu'on augmente une sollicitation du plateau (4) en direction du corps (12).

8. Prothèse selon la revendication 7, **caractérisée en ce que** la zone de contact est définie par une face du plateau et une face de;l'extrémité du corps, l'une (18) des deux faces (18, 20) étant courbe et convexe et l'autre face (20) étant plane.

9. Prothèse selon la revendication 7, **caractérisée en ce que** la zone de contact est définie par une face du plateau et une face de l'extrémité du corps, les deux faces (18, 20) étant courbes dans au moins une direction commune et étant respectivement concave et convexe, la face concave (20) ayant au moins un rayon de courbure supérieur à un rayon de courbure correspondant de la face convexe (18).

10. Prothèse selon l'une quelconque des revendications 1 à 9, **caractérisée en ce que** le corps présente au moins une extrémité en contact avec l'un des plateaux, cette extrémité étant libre de se mouvoir par rapport au plateau suivant une direction parallèle au plateau.

11. Prothèse selon la revendication 10, **caractérisée en ce que** l'extrémité (18) est logée dans un renfoncement (32) du plateau (4) et apte à former une butée latérale pour le corps.

12. Prothèse selon l'une quelconque des revendications 1 à 11, **caractérisée en ce que** le coussin (10) comporte une enceinte (22) renfermant le fluide et agencée de sorte qu'elle a une superficie de section transversale parallèlement aux plateaux (4) sensiblement invariable lorsque varie une compression du coussin entre les plateaux.

13. Prothèse selon l'une quelconque des revendications 1 à 12, **caractérisée en ce que** le coussin (10) comporte une enceinte (22) renfermant le fluide et s'étendant en périphérie et à distance du corps.

14. Prothèse selon la revendication 12 ou 13, **caractérisée en ce que** l'enceinte (22) forme ressort.

15. Prothèse selon l'une quelconque des revendications 1 à 14, **caractérisée en ce que** le coussin (10) est agencé pour présenter une courbe (C) de réaction mécanique (R) à une compression (F) en fonction d'une variation (Al) d'une dimension (1) du coussin (10) suivant la direction (14) de la compression (F), ayant une forme en hystérésis.

16. Prothèse selon la revendication 15, **caractérisée en ce que** le coussin (10) est agencé de sorte que la réaction (R) à la compression (F) croît moins fortement pour des valeurs (Ca1) de réaction (R) relativement faibles que pour des valeurs de réaction (R) relativement élevées (Ca2).

17. Prothèse selon l'une quelconque des revendications 15 à 16, **caractérisée en ce que** le coussin (10) est agencé de sorte que la réaction à la compression (F) décroît plus fortement pour des valeurs de réaction (R) relativement élevées (Cd1) que pour des valeurs de réaction (R) relativement faibles (Cd2).

18. Prothèse selon l'une quelconque des revendications 15 à 17, **caractérisée en ce que** le coussin (10) est agencé de sorte que la réaction (R) à la compression (F) a des valeurs plus élevées lorsqu'elle croît (Ca) que lorsqu'elle décroît (Cd).

19. Prothèse selon l'une quelconque des revendications 1 à 18, **caractérisée en ce qu'**il s'agit d'une prothèse de disque intervertébral lombaire.

## Claims

1. Intervertebral disc prosthesis (2), comprising two plates (4) and a cushion (10) interposed between the plates, the cushion comprising a compressible solid body (12) and containing a compressible fluid, **characterized in that** the fluid extends round about the body (12).

2. Prosthesis according to Claim 1, **characterized in that** the fluid has a pressure such that it is more compressible than the body.

3. Prosthesis according to Claim 1 or 2, **characterized in that** the fluid comprises a gas.

4. Prosthesis according to any one of Claims 1 to 3, **characterized in that** the cushion (10) is arranged such that a fluid pressure is applied directly to the plates (4).

5. Prosthesis according to any one of Claims 1 to 4, **characterized in that** the body (12) comprises a viscoelastic material.

6. Prosthesis according to Claim 5, **characterized in that** the material is silicone.

7. Prosthesis according to any one of Claims 1 to 6, **characterized in that** the body (12) has at least one end (18) having a contact zone with one of the plates (4), the prosthesis being arranged such that the contact zone has a surface area which increases whenever a stressing of the plate (4) in the direction of the body (12) is increased.

8. Prosthesis according to Claim 7, **characterized in that** the contact zone is defined by a face of the plate and an end face of the body, one (18) of the two faces (18, 20) being curved and convex and the other face (20) being flat.

9. Prosthesis according to Claim 7, **characterized in that** the contact zone is defined by a face of the plate and an end face of the body, the two faces (18, 20) being curved in at least one common direction and being respectively concave and convex, the concave face (20) having at least one radius of curvature greater than a corresponding radius of curvature of the convex face (18).

10. Prosthesis according to any one of Claims 1 to 9, **characterized in that** the body has at least one end in contact with one of the plates, this end being free to move relative to the plate in a direction parallel to the plate.

11. Prosthesis according to Claim 10, **characterized in that** the end (18) is accommodated in a recess (32) of the plate (4) and apt to form a lateral stop for the body.

12. Prosthesis according to any one of Claims 1 to 11, **characterized in that** the cushion (10) comprises a shell (22) containing the fluid and arranged such that it has a cross-sectional area parallel to the plates (4) which is essentially invariable when variation occurs in a compression of the cushion between the plates.

13. Prosthesis according to any one of Claims 1 to 12, **characterized in that** the cushion (10) comprises a shell (22) containing the fluid and extending round about and at a distance from the body.

14. Prosthesis according to Claim 12 or 13, **characterized in that** the shell (22) forms a spring.

15. Prosthesis according to any one of Claims 1 to 14, **characterized in that** the cushion (10) is arranged to exhibit a hysteresis-shaped curve (C) of mechanical reaction (R) to a compression (F) as a function of a variation (Δ1) in a dimension (1) of the cushion (10) in the direction (14) of the compression (F).

16. Prosthesis according to Claim 15, **characterized in that** the cushion (10) is arranged such that the reaction (R) to the compression (F) grows less markedly for relatively low reaction (R) values (Ca1) than for relatively high reaction (R) values (Ca2).

17. Prosthesis according to either of Claims 15 and 16, **characterized in that** the cushion (10) is arranged such that the reaction to the compression (F) diminishes more markedly for relatively high reaction (R) values (Cd1) than for relatively low reaction (R) values (Cd2).

18. Prosthesis according to any one of Claims 15 to 17, **characterized in that** the cushion (10) is arranged such that the reaction (R) to the compression (F) has higher values when it grows (Ca) than when it diminishes (Cd).

19. Prosthesis according to any one of Claims 1 to 18, **characterized in that** the prosthesis in question is a lumbar intervertebral disc prosthesis.

## Patentansprüche

1. Bandscheibenprothese (2), die zwei Platten (4) und ein zwischen den Platten sitzendes Kissen (10) umfaßt, wobei das Kissen einen kompressiblen festen Korpus (12) umfaßt und ein kompressibles Fluid einschließt, **dadurch gekennzeichnet, daß** das Fluid sich am Umfang des Korpus (12) erstreckt.

2. Prothese nach Anspruch 1, **dadurch gekennzeichnet, daß** das Fluid unter einem solchen Druck steht, daß es kompressibler ist als der Korpus.

3. Prothese nach Anspruch 1 oder 2, **dadurch gekennzeichnet, daß** das Fluid ein Gas umfaßt.

4. Prothese nach einem der Ansprüche 1 bis 3, **dadurch gekennzeichnet, daß** das Kissen (10) so ausgebildet ist, daß eine Druckkraft des Fluids direkt auf die Platten (4) einwirkt.

5. Prothese nach einem der Ansprüche 1 bis 4, **dadurch gekennzeichnet, daß** der Korpus (12) ein viskoelastisches Material umfaßt.

6. Prothese nach Anspruch 5, **dadurch gekennzeichnet, daß** das Material Silikon ist.

7. Prothese nach einem der Ansprüche 1 bis 6, **dadurch gekennzeichnet, daß** der Korpus (12) wenigstens ein Ende (18) aufweist, das eine Kontaktzone mit einer der Platten (4) hat, wobei die Prothese so ausgebildet ist, daß die Kontaktzone eine Fläche hat, die sich vergrößert, wenn eine Beaufschlagung der Platte (4) in Richtung des Korpus (12) vergrößert wird. '

8. Prothese nach Anspruch 7, **dadurch gekennzeichnet, daß** die Kontaktzone durch eine Seite der Platte und eine Seite des Korpusendes definiert ist, wobei die eine (18) der beiden Seiten (18, 20) gekrümmt und konvex ist und die andere Seite (20) eben ist.

9. Prothese nach Anspruch 7, **dadurch gekennzeichnet, daß** die Kontaktzone durch eine Seite der Platte und eine Seite des Korpusendes definiert ist, wobei die beiden Seiten (18, 20) in wenigstens einer gemeinsamen Richtung gekrümmt sind und konkav bzw. konvex sind, wobei die konkave Seite (20) wenigstens einen größeren Krümmungsradius hat als ein entsprechender Krümmungsradius der konvexen Seite (18).

10. Prothese nach einem der Ansprüche 1 bis 9, **dadurch gekennzeichnet, daß** der Korpus wenigstens ein Ende hat, das in Kontakt mit einer der Platten ist, wobei dieses Ende entlang einer Richtung parallel zur Platte frei beweglich in Bezug auf die Platte ist.

11. Prothese nach Anspruch 10, **dadurch gekennzeichnet, daß** das Ende (18) sich in einer Vertiefung (32) der Platte (4) befindet, die dafür eingerichtet ist, für den Korpus einen seitlichen Anschlag zu bilden.

12. Prothese nach einem der Ansprüche 1 bis 11, **dadurch gekennzeichnet, daß** das Kissen (10) eine Einfassung (22) aufweist, die das Fluid einschließt und so ausgebildet ist, daß sie eine im wesentlichen unveränderliche Querschnittsfläche parallel zu den Platten (4) hat, wenn eine Kompression des Kissens zwischen den Platten variiert.

13. Prothese nach einem der Ansprüche 1 bis 12, **dadurch gekennzeichnet, daß** das Kissen (10) eine Einfassung (22) aufweist, die das Fluid einschließt und die sich am Umfang und in einem Abstand vom Korpus erstreckt.

14. Prothese nach Anspruch 12 oder 13, **dadurch gekennzeichnet, daß** die Einfassung (22) eine Feder bildet.

15. Prothese nach einem der Ansprüche 1 bis 14, **dadurch gekennzeichnet, daß** das Kissen (10) so ausgebildet ist, daß es eine Kurve (C) der mechanischen Reaktion (R) auf eine Kompression (F) in Abhängigkeit von einer Veränderung (ΔI) einer Abmessung (I) des Kissens (10) in der Richtung (14) der Kompression (F) aufweist, die eine i-iystereseform hat.

16. Prothese nach Anspruch 15, **dadurch gekennzeichnet, daß** das Kissen (10) so ausgebildet ist, daß die Reaktion (R) auf die Kompression (F) für relativ kleine Werte (Ca1) der Reaktion (R) weniger stark zunimmt als für relativ hohe (Ca2) Werte der Reaktion (R).

17. Prothese nach einem der Ansprüche 15 bis 16, **dadurch gekennzeichnet, daß** das Kissen (10) so ausgebildet ist, daß die Reaktion auf die Kompression (F) für relativ hohe (Cd1) Werte der Reaktion (R) stärker abnimmt als für relativ kleine (Cd2) Werte der Reaktion (R).

18. Prothese nach einem der Ansprüche 15 bis 17, **dadurch gekennzeichnet, daß** das Kissen (10) so ausgebildet ist, daß die Reaktion (R) auf die Kompression (F) höhere Werte hat, wenn sie zunimmt (Ca) als wenn sie abnimmt (Cd).

19. Prothese nach einem der Ansprüche 1 bis 18, **dadurch gekennzeichnet, daß** es sich um eine Lendenwirbel-Bandscheibenprothese handelt.
